# EUROPEAN PATENT SPECIFICATION

(11) **EP 4 495 488 B1**
(45) Date of publication and mention of the grant of the patent: **09.12.2026**
(21) Application number: 23902326.0
(22) Date of filing: 31.10.2023
(51) Int. Cl.: F24C 15/20, F24F 3/16, F24C 7/00, H05B 6/12

(54) **OIL SMOKE EXTRACTION DEVICE, AND INDUCTION HOB INTEGRATED COOKER**
ÖLRAUCHEXTRAKTIONSVORRICHTUNG UND INDUKTIONSKOCHFELDINTEGRIERTER KOCHER
DISPOSITIF D'EXTRACTION DE FUMÉE D'HUILE ET CUISEUR INTÉGRÉ À PLAQUE À INDUCTION

(30) Priority: 16.12.2022 CN 202211622062
(43) Date of publication of application: 22.01.2025
(73) Proprietor: Guangdong Arcair Appliance Co., Ltd., Guangdong 528318 (CN)
(72) Inventor: KANG, Zuotian, Foshan, Guangdong 528318 (CN); TANG, Haijiang, Foshan, Guangdong 528318 (CN); LIANG, Haisheng, Foshan, Guangdong 528318 (CN)
(74) Representative: Murgitroyd & Company
(86) International application number: PCT/CN2023/128146
(87) International publication number: WO 2024/125126

(56) References cited:
- EP-A2- 3 511 633
- CN-A- 111 720 868
- CN-A- 111 720 868
- CN-A- 115 468 203
- CN-A- 115 468 203
- CN-A- 115 962 501
- CN-U- 203 586 315
- CN-U- 217 503 768
- DE-A1- 102019 101 060
- US-A1- 2013 333 685
- US-A1- 2021 215 350
- US-A1- 2021 215 350

## Description

### TECHNICAL FIELD

The present invention relates to the field of range hoods and in particular to a fume extracting device and an induction cooker integrated stove.

### BACKGROUND

At present, the range hoods on the markets are mounted above countertop to suck fume into the range hoods from bottom up. Such range hoods have a large volume and also need a large ventilation pipe cooperating with an exhaust fan. For this reason, the entire structure occupies a large space, and cannot be applied to the environments with relatively small mounting spaces. Further, these range hoods have large noise. Therefore, it is necessary to make improvements.

The following documents of the prior art are considered to be relevant to the present invention: US 2021/215350 A1; CN 115468203 A; CN 111720 868 A; DE 102019101060 A1; EP 3511633 A2.

### SUMMARY

The present invention aims to solve, at least to some degree, one of the above technical problems in the related arts. For this purpose, the present invention provides a fume extracting device and an induction cooker integrated stove.

According to a first aspect of the invention, there is provided a fume extracting device, which includes:
a box body, wherein a mounting hole is disposed on an upper end surface of the box body;
a volute fan, located inside the box body, wherein an air inlet end in a middle part of the volute fan faces downward;
a fume extracting pipe, located inside the box body, wherein one end of the fume extracting pipe is inserted upward through the mounting hole and disposed on the box body, and the other end of the fume extracting pipe is in communication with the air inlet end of the volute fan; the end of the fume extracting pipe inserted through the box body extends horizontally to form a support base, a lower side of the support base is abutted against and fixed on the upper end surface of the box body, and a filtering assembly is disposed inside the support base to filter gases entering the fume extracting pipe from outside; and
a fume exhaust pipe, wherein the fume exhaust pipe is in communication with an air outlet end of the volute fan.

The fume extracting device in the embodiments of the present invention at least has the following technical effects: the fume extracting device is mounted under countertop, occupying small space. Further, the air inlet end of the volute fan faces downward, leading to noise reduction.

According to some embodiments of the present invention, the filtering assembly includes a filtering cup, a strainer and an air inlet cover. A part of structure of the filtering cup is inserted through the mounting hole into the fume extracting pipe, and a part of structure of the filtering cup is abutted against the support base to enable the support base to support the filtering cup. An opening of an upper end of the filtering cup matches an opening of an upper end of the support base. The strainer is laid on the opening of the upper end of the filtering cup, and the air inlet cover is covered on the strainer.

According to some embodiments of the present invention, the filtering cup includes a support cradle and a filtering cartridge, and a side of the support cradle is in communication with an upper end of the filtering cartridge. A lower end of the support cradle is abutted against the support base and the filtering cartridge is inserted into the fume extracting pipe.

According to some embodiments of the present invention, lift handles are disposed at the bottom of the support cradle.

According to some embodiments of the present invention, the fume extracting pipe further includes a first pipe and a second pipe. The first pipe extends vertically. An upper end of the first pipe is connected to the support base and a lower end of the first pipe is connected to one end of the second pipe and another end of the second pipe is connected with the air inlet end of the volute fan.

According to some embodiments of the present invention, a water outlet hole is disposed at the bottom of an inner chamber of the second pipe, and an air outlet hole is disposed on the top of the inner chamber of the second pipe; the water outlet hole and the air outlet hole are coaxially disposed.

According to some embodiments of the present invention, the bottom of the inner chamber of the second pipe is gradually recessed downwardly in a direction of being close to the water outlet hole. The fume extracting device further includes a rotary plug detachably connected with the second pipe to close the water outlet hole.

According to some embodiments of the present invention, a sidewall of the filtering cartridge is provided with an air vent to enable gases to enter the first pipe from the filtering cartridge.

According to some embodiments of the present invention, the volute fan includes a volute, a wind wheel and a motor. The volute is mounted above the second pipe and abutted against a sidewall of the second pipe. The wind wheel is located inside the volute and the motor is located above the volute. A motor shaft of the motor faces downward to connect with the wind wheel.

According to a second aspect of the present invention, there is provided an induction cooker integrated stove, which includes the fume extracting device mentioned in any of the above-described embodiments.

The induction cooker integrated stove in the embodiments of the present invention at least has the following technical effects: the induction cooker integrated stove uses the fume extracting device, which occupies small space. Further, the air inlet end of the volute fan faces downward, leading to noise reduction.

Additional aspects and advantages of the present invention will be partially given in the following descriptions and partially become obvious from the following descriptions or understood by practice of the present disclosure.

### BRIEF DESCRIPTION OF DRAWINGS

The above and/or additional aspects and advantages of the present disclosure will become obvious and intelligible from the descriptions made to the embodiments by referring to the drawings.
FIG. 1 is a structural schematic diagram illustrating a fume extracting device according to some embodiments of the present disclosure.
FIG. 2 is a schematic diagram illustrating a partial structure of a fume extracting device according to some embodiments of the present disclosure.
FIG. 3 is a structural schematic diagram illustrating a box body according to some embodiments of the present disclosure.
FIG. 4 is a structural schematic diagram illustrating a fume extracting pipe according to some embodiments of the present disclosure.
FIG. 5 is an exploded view of a partial structure of a fume extracting device according to some embodiments of the present disclosure.
FIG. 6 is a structural schematic diagram illustrating a filtering cup according to some embodiments of the present disclosure.
FIG. 7 is a sectional view of a filtering cup according to some embodiments of the present disclosure.
FIG. 8 is a schematic diagram illustrating a partial structure of a fume extracting device according to some embodiments of the present disclosure.
FIG. 9 is an efficiency diagram illustrating a fume extracting device according to some embodiments of the present disclosure.

The numerals of the drawings are described below:
100. box body, 110. mounting hole;
200. volute fan;
300. fume extracting pipe, 310. support base, 320. first pipe, 330. second pipe, 331. water outlet hole, 332. rotary plug, 333. air outlet hole;
400. filtering assembly, 410. filtering cup, 411. support cradle, 412. filtering cartridge, 413. lift handle, 414. air vent, 415. water reservoir, 420. strainer, 430. air inlet cover, 440. fastening strip;
500. fume exhaust pipe.

### DETAILED DESCRIPTION

The embodiments of the present disclosure will be detailed below with examples illustrated in the accompanying drawings. Same or similar numerals represent same or similar elements or elements having same or similar functions throughout the specification. The embodiments described below by referring to the drawings are only illustrative, and used to explain the present disclosure and shall not be understood as limiting of the present disclosure.

In the descriptions of the present disclosure, it should be understood that in the orientation descriptions, the orientation or position relationship indicated by the terms such as upper, lower, front, rear, left and right and the like is based on the orientation or position relationship shown in the drawings and is used only to help the description of the present disclosure and simplify the descriptions rather than to indicate or imply that the indicated device or element must have a specific orientation or be constructed or operated at a specific orientation and thus shall not be understood as limiting of the present disclosure.

In the descriptions of the present disclosure, the term "plural" means two or more, the terms "greater than", "less than" "more than" and the like shall not be understood as including the present number, and the terms "above", "below" and "within" and the like shall be understood as including the present number. The descriptions of the first and the second are used only to distinguish technical features and shall not be understood as indicating or implying any relative importance, or implicitly indicating the number of the indicated technical features or implicitly indicating precedence of the indicated technical features.

In the descriptions of the present disclosure, unless otherwise clearly stated, the terms "dispose", "mount" and "connect" and the like shall be understood in broad sense, and those skilled in the arts can reasonably determine the specific meanings of the above technical terms in the present disclosure based on the specific contents of the technical solutions.

The present disclosure will be further described below in combination with the drawings.

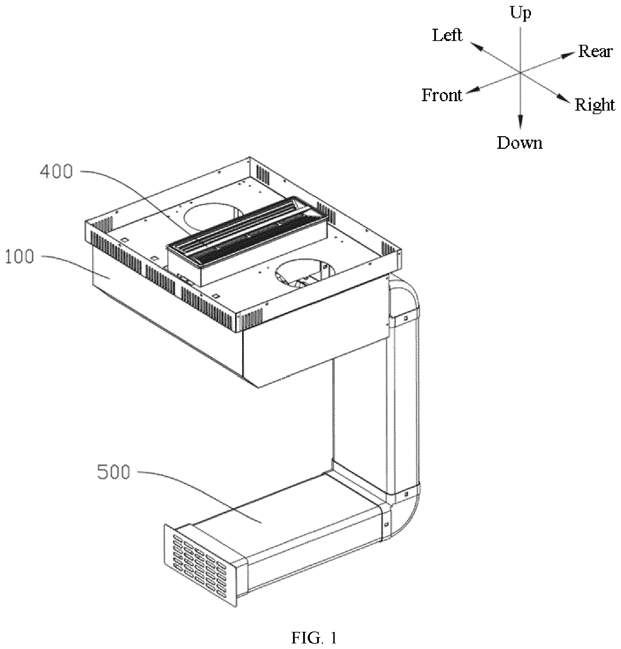

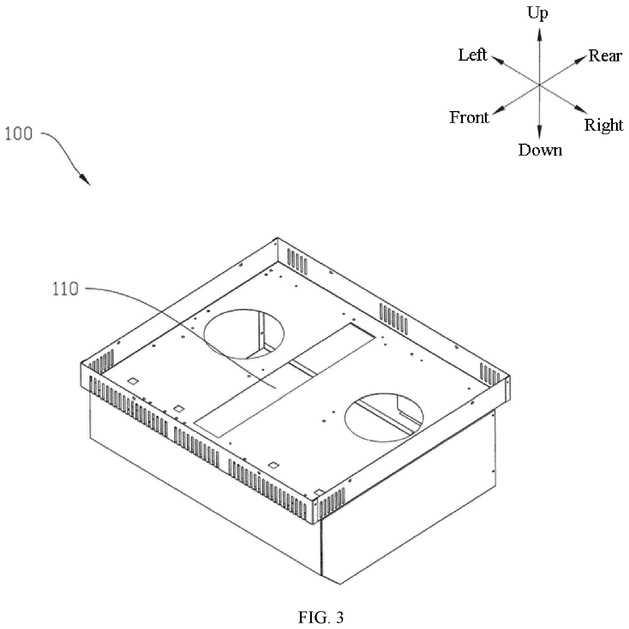

According to some embodiments of the present disclosure, as shown in FIGS. 1 to 7, there is provided a fume extracting device including a box body 100, a volute fan 200, a fume extracting pipe 300 and a fume exhaust pipe 500. A mounting hole 110 is disposed on an upper end surface of the box body 100. The volute fan 200 is located inside the box body 100, with its air inlet end in a middle part of the volute fan 200 facing downward. The fume extracting pipe 300 is located inside the box body 100. One end of the fume extracting pipe 300 is inserted upward through the mounting hole 110 and disposed on the box body 100, and the other end of the fume extracting pipe 300 is in communication with the air inlet end of the volute fan 200. The fume exhaust pipe 500 is in communication with an air outlet end of the volute fan 200.

The conventional side suction type range hoods have the disadvantages of high noise, high energy consumption and frequent cleaning. The reason why the working noise of the side suction type range hoods is much higher includes many factors such as internal design, external shape, and motor working mode of the side suction type range hoods, which is highlighted especially at the high-speed level of the side suction type range hoods. Since the side suction type range hoods are mounted close to a cooking stove, the working heat of the cooking stove can be lost obviously, leading to increased gas consumption. Furthermore, since the side suction type range hoods are close to a cooking stove or a wok, vegetable soups or oils may be splashed more easily to the side suction type range hoods during home cooking process. Therefore, once dish cooking is finished, it is required to immediately clean the surfaces of the side suction type range hoods in order to keep kitchen clean. The cleaning work of the side suction type range hoods should be carried out more thoroughly and more frequently. Because the side suction type range hoods occupy a large wall surface space, the cooking space can be significantly occupied. This disadvantage is especially obvious for those early models of side suction type range hoods.

In this embodiment, the fume extracting device is mounted under countertop, occupying small space. Further, the air inlet end of the volute fan 200 faces downward, leading to noise reduction. In addition, a partial structure of the box body 100 serves as an end of the fume extracting pipe 300. Compared with the conventional side suction type range hoods, a filtering assembly 400 is mounted on a plane of the box body 300, and an area to be cleaned is small and thus vegetable soups and oils will not be easily splashed to an air inlet opening of the fume extracting pipe 300. Further, the filtering assembly 400 can be easily dismounted for cleaning. Furthermore, the fume extracting device in this embodiment doe not tend to suck the working heat generated by the cooking stove, reducing the gas consumption.

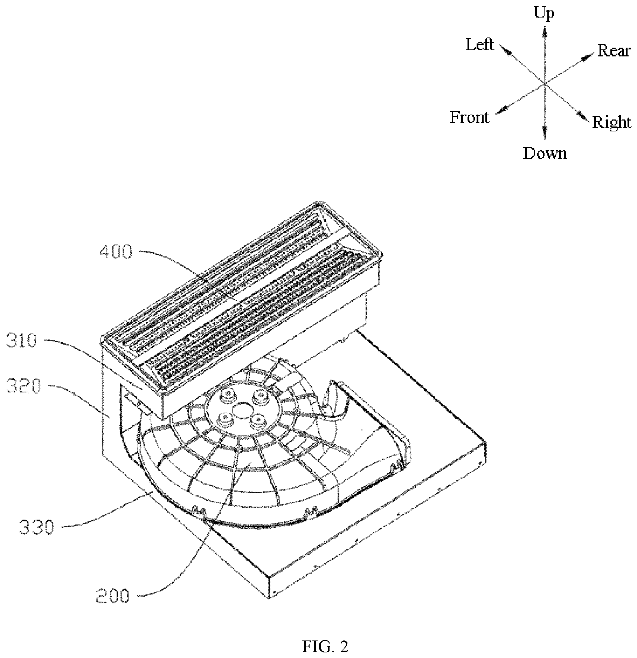

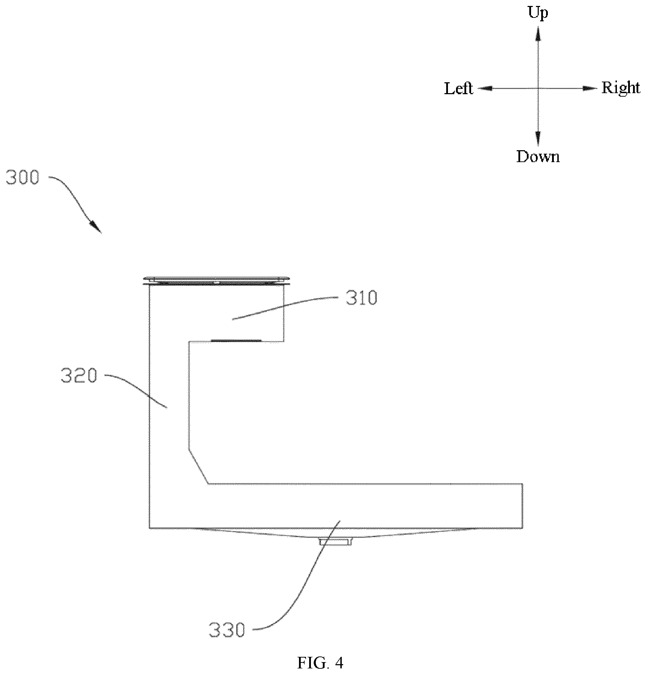

According to some embodiments of the present disclosure, as shown in FIGS. 2 and 4, the end of the fume extracting pipe 300 inserted through the box body 100 extends horizontally to form a support base 310, namely, an upper end of the fume extracting pipe 300 extends rightward to form the support base 310. With reference to FIGS. 1 to 3, the upper end of the fume extracting pipe 300 runs through the mounting hole 110 and a lower side of the support base 310 is abutted against and fixed on the upper end surface of the box body 100, and hence the fume extracting pipe 300 can be fixed on the box body 100. The filtering assembly 400 is disposed inside the support base 310 to filter gases entering the fume extracting pipe 300 from outside.

It should be noted that the fume extracting pipe 300 in this embodiment has a smaller fume-extracting opening and can generate a larger suction force under same power compared with conventional range hoods.

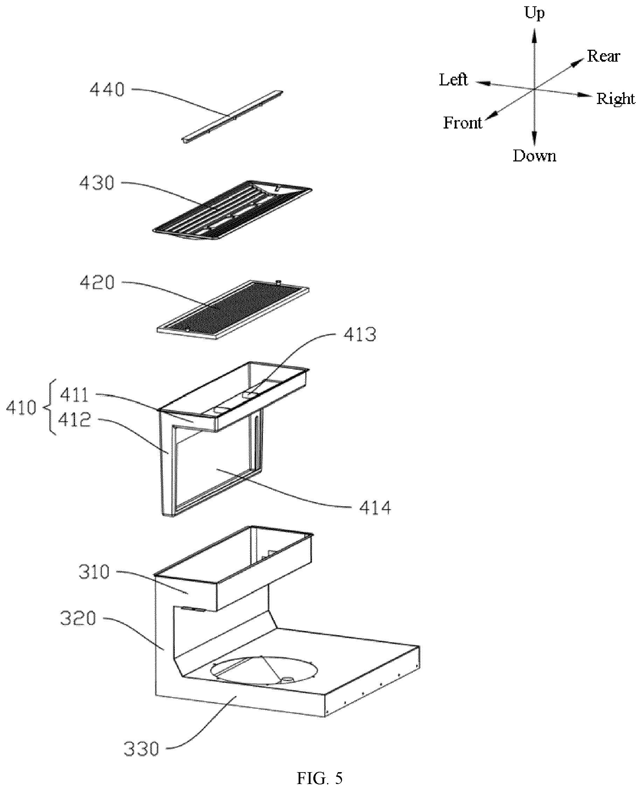

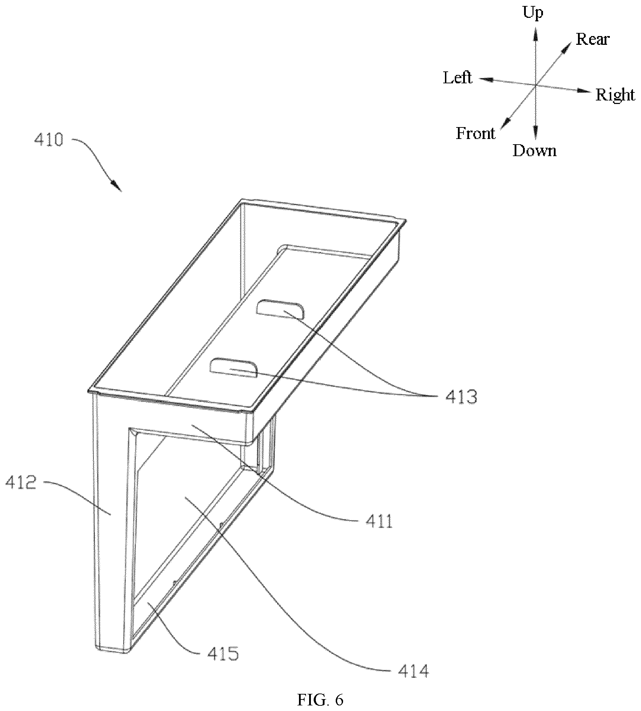

According to some embodiments of the present disclosure, as shown in FIGS. 5 and 6, the filtering assembly 400 includes a filtering cup 410, a strainer 420 and an air inlet cover 430. A part of structure of the filtering cup 410 is inserted through the mounting hole 110 into the fume extracting pipe 300, and the other part of structure of the filtering cup 410 is abutted against the support base 310 to enable the support base 310 to support the filtering cup 410. An opening of an upper end of the filtering cup 410 matches an opening of an upper end of the support base 310. The strainer 420 is laid on the opening of the upper end of the filtering cup 410, and the air inlet cover 430 is covered on the strainer 420.

Preferably, as shown in FIGS. 5 to 7, the filtering cup 410 includes a support cradle 411 and a filtering cartridge 412, and a left side of the support cradle 411 is in communication with an upper end of the filtering cartridge 412. A lower end of the support cradle 411 is abutted against the support base 310 such that the support base 310 can support the filtering cup 410. The filtering cartridge 412 is matched in shape with the fume extracting pipe and inserted into the fume extracting pipe 300. In this embodiment, the filtering cartridge 412 is a cuboid, and a right sidewall of the filtering cartridge is provided with an air vent 414 to enable gases to enter the fume extracting pipe 300 from the filtering cartridge 412.

When the volute fan 200 works, external gas enters the strainer 420 through the air inlet cover 430 to filter out impurities such as oils and then enters the filtering cup 410 and then enters the fume extracting pipe 300 from the filtering cup 410, and then enters the fume exhaust pipe 500 through the volute fan 200 for discharge.

Preferably, as shown in FIG. 6, lift handles 413 are disposed at the bottom of the support cradle 411. It can be understood that since the filtering cup 410 needs to be cleaned regularly, the lift handles 413 can help take out the filtering cup 410 from down up; after the filtering cup 410 is cleaned, the filtering cup 410 can be then placed into the fume extracting pipe 300 from top down.

Preferably, as shown in FIG. 5, a fastening strip 440 is disposed above the air inlet cover 430 to fix the air inlet cover 430 to the support base 310, and hence, the filtering assembly 400 can be fixed on the support base 310.

According to some embodiments of the present disclosure, as shown in FIGS. 4 and 5, the fume extracting pipe 300 further includes a first pipe 320 and a second pipe 330. The first pipe 320 extends vertically. An upper end of the first pipe 320 is connected to the support base 310 and a lower end of the first pipe 320 is connected to one end of the second pipe 330 and another end of the second pipe 330 is connected with the air inlet end of the volute fan 200.

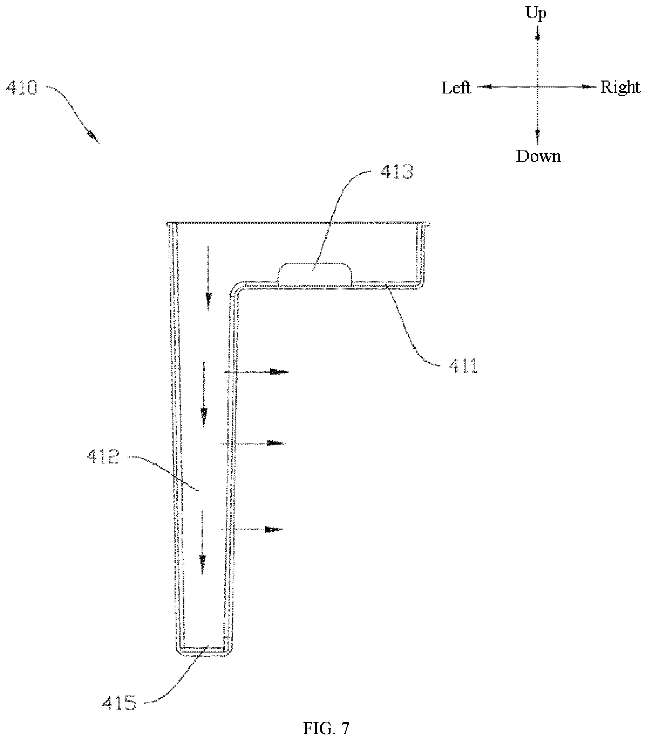

Preferably, as shown in FIGS. 6 and 7, the arrow direction in FIG. 7 refers to an air flow direction, and the air vent 414 is disposed on the right sidewall of the filtering cartridge 412 such that the gas can enter the first pipe 320 from the filtering cartridge 412. In this embodiment, the air vent 414 on the right sidewall of the filtering cartridge 412 is large, which helps the gas in the filtering cartridge 412 to run through the air vent 414 at a large flow rate, reducing the influence of the filtering cartridge 412 on the gas flow efficiency. Furthermore, when the filtering cartridge 412 is to be cleaned, an inner chamber of the filtering cartridge 412 can be cleaned through the air vent 414.

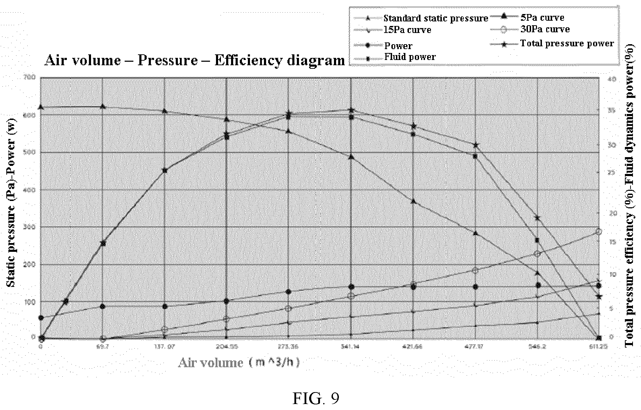

It should be noted that, based on the fluid energy conservation equation $P + \frac{1}{2} {ρv}^{2} + ρgh = C$ (constant), where P is a static pressure, $\frac{1}{2} {ρv}^{2}$ is a dynamic pressure, and ρgh is a weight, it can be known that when a gas flow rate increases with a decreasing pressure to enable the gas flow rate at a minimum section of the filtering cartridge 412 to reach maximum and the high-speed gas flow enters the volute fan 200 through the second pipe 330, a negative pressure area is formed at the bottom of the filtering cartridge 412. Due to the suction effect formed in the negative pressure area, the fume carried by the gas flowing through the filtering cartridge 412 is sucked to the bottom of the filtering cartridge 412 to prevent the fume from entering the volute fan 200 to affect the performance of the volute fan 200. The fume extracting device has the advantages of low energy consumption, good fume extracting effect and protection of the volute fan 200 from fume contamination. The fume extracting pipe 300 of the fume extracting device in this embodiment has the efficiencies shown in FIG. 9 under different powers.

It should be further noted that the efficiencies of the fume extracting pipe 300 of the fume extracting device under different powers in this embodiment are shown in FIG. 9 and Table 1, where Table 1 shows a performance test parameter table of a fume extracting device in this embodiment. When the power of the fume extracting device increases gradually, the standard static pressure gradually decreases, and the fluid efficiency and the total pressure efficiency both firstly increase and then decrease. Based on FIG. 9 and Table 1, the fume extracting device can be adjusted to an optimal power to generate an optimal working efficiency, and therefore the fume extracting device in this embodiment has the advantages of low energy consumption, good gas suction effect and high efficiency.

**Table 1**

| NO. | Air Flux | Static Pressure | Standard Static Pressure | Standard Total Pressure | FDEhood | Power | Total Efficiency |
|---|---|---|---|---|---|---|---|
| | m3/h | Pa | Pa | Pa | % | W | % |
| 1 | 0 | 592.58 | 623.56 | 623.56 | 0 | 78.96 | 0 |
| 2 | 69.7 | 599.19 | 630.52 | 631.24 | 13.99 | 87.28 | 14 |
| 3 | 137.07 | 577.92 | 608.14 | 610.93 | 25.36 | 91.3 | 25.48 |
| 4 | 204.55 | 546.19 | 574.75 | 580.98 | 31.61 | 103.32 | 31.95 |
| 5 | 273.36 | 511.95 | 538.71 | 549.84 | 33.95 | 120.49 | 34.65 |
| 6 | 341.14 | 461.47 | 485.6 | 502.92 | 33.92 | 135.64 | 35.14 |
| 7 | 421.66 | 349.34 | 367.61 | 394.08 | 30.23 | 142.44 | 32.41 |
| 8 | 477.17 | 268.93 | 283 | 316.9 | 26.42 | 142 | 29.58 |
| 9 | 546.2 | 131.83 | 138.72 | 183.14 | 14.76 | 142.58 | 19.49 |
| 10 | 611.25 | 0 | 0 | 55.63 | 0 | 142.93 | 6.61 |

According to some embodiments of the present disclosure, a purifying device including an ozone generator and a UV disinfection assembly is disposed between the fume exhaust pipe 500 and the air outlet end of the volute fan 200. When external gas enters the purifying device through the air outlet end of the volute fan 200, the gas reacts with ozone generated by the ozone generator so as to perform sterilization and deodorization. Next, the UV disinfection assembly sends ultraviolet rays to perform further sterilization on the gas and decompose redundant ozone. After the above purification process, the gas is discharged through the fume exhaust pipe 500.

It can be understood that after the gas runs through the filtering assembly 400 and then enters the volute fan 200 through the fume extracting pipe 300, the existing range hoods will discharge the gas directly together with impurities and bacteria not filtered out by the filtering assembly 400. Therefore, the purifying device is needed to perform further purification on the gas before discharging the gas, avoiding atmospheric pollution.

The ozone can carry out oxidative decomposition on enzymes desired for glucose decomposition in bacteria to disable tricarboxylic acid (TCA) cycle and hence disable the supply of adenosine triphosphate (ATP) desired by cell life activity, leading to bacteria inactivation and death. Ozone is an efficient broad-spectrum sterilizing agent which can have extremely strong killing effect on bacteria, viruses, molds, spores, fungi, and microbes. The ozone has a sterilization ability 20% higher than that of ultraviolet rays, and can spontaneously diffuse in the air to achieve full sterilization. Further, the ozone has a fast sterilizing speed in water, 600 times faster than chlorine. It completes its mechanism for virus inactivation by directly destroying the RNA or DNA substance of the viruses, and when killing bacteria and mold microbes, the ozone firstly acts on cell nucleus and then destroys intra-membranous tissues until killing them. The oxidation ability of the ozone is double that of chlorine, and the sterilization speed of the ozone is 600 to 3000 times that of chlorine and thus it can kill bacteria even in several seconds. Further, the ozone is a green and environment-friendly element and can spontaneously reduce to oxygen and water during sterilization and disinfection process, without leaving any residues and generating secondary pollution. The ozone can kill vegetative bacteria, spores and fungi, destroy botulinum fungi and the like, remove and kill toxic substances and bacteria in the air, water and foods, and remove odors. Those common bacillus coli, streptococcus, pseudomonas aeruginosa, staphylococcus aureus, toadstool and the like can be killed at the rate of above 99% in ozone environment in15 minutes. The ozone generates non-toxic and odorless substances by destroying molecular structures generating odorous substances. Since the phased oxygen (nascent oxygen) generated during ozone decomposition has strong oxidizing property, the odors, toxic gases and the like in the organic pollutants during the decomposition process can be decomposed in short time to generate air and become stable and odorless. The chemical reaction of ozone and formaldehyde is: 2O₃+HCHO→2O₂+H₂O₂+CO₂, and the chemical reaction of ozone and benzene is: C₆H₆+5O₃→6CO₂+3H₂O.

The ultraviolet rays can be divided into four bands based on wavelength: long wave UVA, medium wave UVB, short wave UVC and vacuum wave UVD.

The long wave UVA has a wavelength of 320 to 400 nm and a strong penetration power and can penetrate through glass and even 9 feet of water. It is always present four seasons a year regardless of the fact that it is cloudy or sunny and dusk or dawn. The harm to human body: more than 95% of ultraviolet rays contacted by skin during daily life are UVA and hence it has the most severe harm to the skins. The UVA can penetrate through epidermis to attack dermis, severely harming ossein and elastin in the skins. Since the dermal cells have poor self protection ability, a small number of UVA can cause extremely large harm to them. Over time, the problems such as skin laxity, winkles and high microvascular visibility can occur. Further, it can also activate tyrosinase to bring instant melanosis and new melanin formation, causing the skin to become black and lack luster. The UVA can cause long-time, chronic and permanent harm, making the skin age earlier. Therefore, the UVA is also called aging rays.

UVA application field: the UVA of 360nm wavelength complies with the phototactic reaction curve of insects and therefore can be used for light traps. The UVA of 300 to 420nm wavelength can fully penetrate through special tinted glass tubes completely cutting off visible light and only irradiate out near ultraviolet light with 365nm as center and thus can be used for ore identification, stage decoration and counterfeit currency detection and the like.

The medium wave UVB has a wavelength of 275 to 320nm and a medium penetration power. The short parts of its wavelength can be absorbed by transparent glass, and the medium-wavelength ultraviolet rays in the day light are mostly absorbed by the ozone layer, with only less than 2% reaching the surface of the earth, which will become much stronger in summer and in afternoon. The harm to human body: it can oxidize the protective lipid layer of the epidermis, drying the skin; further, nucleic acid and proteins in the epidermic cells are modified, generating acute dermatitis (sunburn) and the like, and making the skin red and painful. In severe cases, for example, long-time insolation can also cause skin canceration. Furthermore, the long-time harm of the UVB can also cause variation of the melanophores, leading to incurable solar lentigo.

UVB application field: the ultraviolet health lamps and plant growth lamps are made of special ultraviolet-transmission glass (light below 254nm does not transmit through it) and a fluorescent powder with peak value near 300nm.

The short wave UVC has a wavelength of 200 to 275 nm and is also called short-wave sterilizing ultraviolet ray. It has the weakest penetration power and cannot penetrate through most transparent glasses and materials. The short-wave ultraviolet rays in the day light are almost fully absorbed by the ozone layer before reaching the ground. The harm to human body: since the UVC in the nature is absorbed by the ozone layer before reaching the ground, its influence on the skin is negligible. But, because the short-wave ultraviolet rays have large harm to human body, it is not allowed to directly irradiate on human body. In case of direct irradiation, short-time irradiation can burn the skin and long-time or high-intensity irradiation can cause skin cancer.

UVC application field: the ultraviolet ray sterilization lamp emits such UVC short-wave ultraviolet rays. The short-wave ultraviolet rays are widely applied to the fields such as hospitals, air-conditioning systems, disinfection cabinets, water treatment equipment, water dispensers, wastewater treatment plants, swimming pools, foods and beverage processing and packaging equipment, foods factories, cosmetics factories, milk product factories, breweries, beverage factories, bakeries and cold storage rooms and the like.

The bond energy of the ozone is 101.2kJ/mol, and the ozone has a maximal energy absorption at the wavelength of 254nm. After the ozone absorbs the ultraviolet light, the dissociation reaction occurs: O₃+hv→O·+O₂. Under the synergic effect of the ultraviolet light, due to formation of alkyl radicals, the ozone effectively destroys the molecular structures of the organic matters and finally decomposes them: O₃+H₂O+hv→O₂+H₂O₂ , H₂O₂+hv→2·OH.

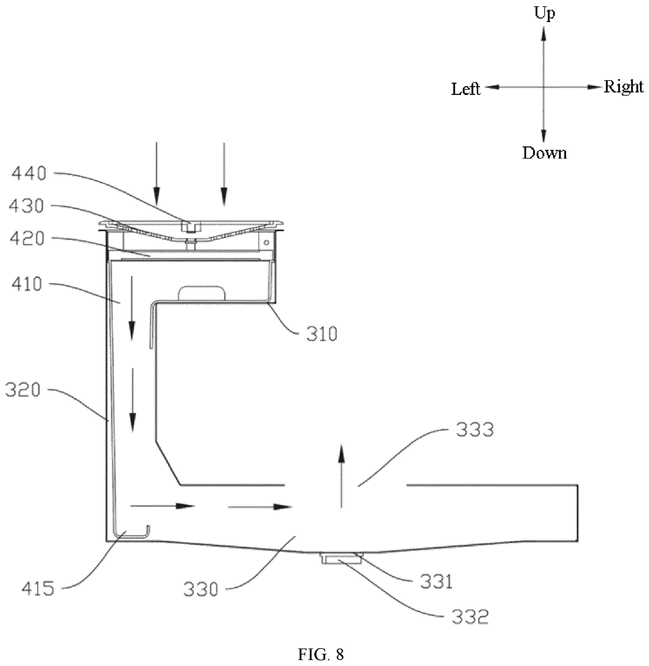

According to some embodiments of the present disclosure, as shown in FIG. 8, a water outlet hole 331 is disposed at the bottom of an inner chamber of the second pipe 330, and an air outlet hole 333 is disposed on the top of the inner chamber of the second pipe 330; the water outlet hole 331 and the air outlet hole 333 are coaxially disposed, and an axis direction of the water outlet hole 331 and the air outlet hole 333 is up-down direction.

It can be understood that a water reservoir 415 is disposed at the bottom of the inner chamber of the filtering cup 410; when a liquid overflows out of the water reservoir 415, the liquid flows into the second pipe 330, and the liquid in the second pipe 330 can be discharged through the water outlet hole 331.

It can be further understood that, when a gas enters the second pipe 330 through the filtering cup 410, the gas may carry the liquid in the water reservoir 415; when the gas runs upward into the air inlet end of the volute fan 200 through the air outlet hole 333, the liquid carried by the gas falls to the bottom of the second pipe 330 under the action of gravity, preventing the liquid from entering the volute fan 200 to affect the performance of the volute fan 200.

Preferably, with reference to FIG. 8, the bottom of the inner chamber of the second pipe 330 is gradually recessed downwardly in a direction of being close to the water outlet hole 331 such that the liquid in the second pipe 330 moves close to the water outlet hole 331 under the action of gravity, helping the liquid in the second pipe 330 to be discharged through the water outlet hole 331 and avoiding water accumulation in the inner chamber of the second pipe 330. The fume extracting device further includes a rotary plug 332 detachably connected with the second pipe 330 to close the water outlet hole 331. When the liquid in the water reservoir 415 overflows, the liquid enters the second pipe 330 and gradually flows toward the water outlet hole 331, helping liquid collection. In this embodiment, the water outlet hole 331 at the lowest bottom of the inner chamber of the second pipe 330 and the air outlet hole 333 are coaxially disposed, and their axis extends along an up-down direction. Since the gas moves from top down in the filtering cartridge 412, and then moves left to right from the filtering cartridge into the second pipe 330, the gas may carry a small amount of liquid at this time, and the small amount of liquid is separated from the gas under the action of gravity and falls to the bottom of the second pipe 330 and moves close to the water outlet hole 331. In this case, a vertical distance between the liquid and the air outlet hole 333 is larger and larger, and thus the gas is prevented from blowing again the liquid falling into the second pipe 330 into the volute fan 200 and hence affecting the performance of the volute fan 200.

According to some embodiments of the present disclosure, with reference to FIG. 2, the volute fan 200 includes a volute, a wind wheel and a motor. The volute is mounted above the second pipe 330 and fixed on an upper sidewall of the second pipe 330. The wind wheel is located inside the volute and the motor is located above the volute. A motor shaft of the motor faces downward to connect with the wind wheel so as to bring the wind wheel to rotate.

Preferably, the motor is a synchronous motor in which permanent magnet performs excitation to generate synchronous rotating magnetic field. Compared with the conventional motors, the motor has the characteristics of high control accuracy, good adjustment linearity and stable performance. The permanent magnet synchronous motor has no electric brush and no excitation coil and uses permanent magnet material magnetic pole and especially uses rare earth metal permanent magnet (e.g. neodymium iron boron and the like) and can have high air-gap flux density due to high magnetic energy product. Therefore, under same capacity and same power, the permanent magnet synchronous motor, compared with the conventional motors, has the characteristics of small volume, light weight, small heat generation, and low noise, and is more energy-saving and efficient with the energy saving effect far higher than the requirement of the IE standard in force. In this way, the entire volume of the centrifugal circulating fan is reduced while the efficiency is increased. The entire length of the permanent synchronous motor is at least 1/4 shorter than the length of the conventional motor. Thus, the entirety of the fume extracting device is more compact, reducing the occupation space and lowering its basic noise.

Preferably, the wind wheel is a main working component for generating wind pressure and conveying energy. The wind wheel is disposed inside the volute and connected with an output shaft of the permanent magnet synchronous motor. In this embodiment, the transmission mode of the wind wheel and the permanent magnet synchronous motor is type A in which an impeller is fixedly connected with the output shaft of the permanent magnet synchronous motor by impeller locking sheets, impeller locking bolts and radial locking bolts. This transmission mode has the characteristics of compact structure and small volume.

Preferably, the volute has a facing-down air inlet opening and an air outlet opening and is used to direct gases leaving the impellers to the air outlet opening and convert partial dynamic pressure to static pressure to form wind pressure.

Preferably, a flow collector also known as horn mouth is disposed on an upper end of the first pipe 320, and used to suck and collect air flow with smaller resistance and direct the air flow to be uniformly conveyed into the first pipe 320.

When the volute fan 200 operates, the motor brings through the output shaft the wind wheel to rotate at a higher speed than the conventional motor, and a negative pressure area is formed at an air inlet of the flow collector. The gas is conveyed into the filtering assembly 400 under the action of a negative pressure, and filtered by the strainer and then flows into the filtering cup 410, and then sequentially flows through the first pipe 320 and the second pipe 330 and then is sucked into the wind wheel, and rotated along with the wind wheel, and pressurized and then discharged through the air outlet opening of the volute. In this process, when the gas sequentially runs through the first pipe 320 and the second pipe 330, the noise generated by the gas is reduced, and the pneumatic noise, electromagnetic noise and mechanical noise of the fume extracting device are weakened, thereby achieving noise reduction. Further, the air flow scours a housing of the permanent magnet synchronous motor so as to cool the permanent magnet synchronous motor.

Although the embodiments of the present disclosure have been shown and described, persons of ordinary skill in the prior arts may understand that various changes, modifications, replacements and variations can be made to these embodiments without departing from the claims, and the scope of protection of the present invention shall be defined by the claims.

## Claims

1. A fume extracting device, applied to an induction cooker integrated stove, and comprising:
a box body (100), wherein a mounting hole (110) is disposed on an upper end surface of the box body (100);
a volute fan (200), located inside the box body (100), wherein an air inlet end in a middle part of the volute fan (200) faces downward;
a fume exhaust pipe (500), wherein the fume exhaust pipe (500) is in communication with an air outlet end of the volute fan (200);
a fume extracting pipe (300), located inside the box body (100), wherein one end of the fume extracting pipe (300) is inserted upward through the mounting hole (110) and disposed on the box body (100), and the other end of the fume extracting pipe (300) is in communication with the air inlet end of the volute fan (200); **characterized in that** the end of the fume extracting pipe (300) inserted through the box body (100) extends horizontally to form a support base (310), a lower side of the support base (310) is abutted against and fixed on the upper end surface of the box body (100), and a filtering assembly (400) is disposed inside the support base (310) to filter gases entering the fume extracting pipe (300) from outside.

2. The fume extracting device of claim 1, wherein the filtering assembly (400) comprises a filtering cup (410), a strainer (420) and an air inlet cover (430), a part of structure of the filtering cup (410) is inserted through the mounting hole (110) into the fume extracting pipe (300), and a part of structure of the filtering cup (410) is abutted against the support base (310) to enable the support base (310) to support the filtering cup (410), an opening of an upper end of the filtering cup (410) matches an opening of an upper end of the support base (310), the strainer (420) is laid on the opening of the upper end of the filtering cup (410), and the air inlet cover (430) is covered on the strainer (420).

3. The fume extracting device of claim 2, wherein the filtering cup (410) comprises a support cradle (411) and a filtering cartridge (412), and a side of the support cradle (411) is in communication with an upper end of the filtering cartridge (412), a lower end of the support cradle (411) is abutted against the support base (310), and the filtering cartridge (412) is inserted into the fume extracting pipe (300).

4. The fume extracting device of claim 3, wherein lift handles (413) are disposed at the bottom of the support cradle (411).

5. The fume extracting device of claim 3, wherein the fume extracting pipe (300) further comprises a first pipe (320) and a second pipe (330), the first pipe (320) extends vertically, an upper end of the first pipe (320) is connected to the support base (310), a lower end of the first pipe (320) is connected to one end of the second pipe (330), and another end of the second pipe (330) is connected with the air inlet end of the volute fan (200).

6. The fume extracting device of claim 5, wherein a water outlet hole (331) is disposed at the bottom of an inner chamber of the second pipe (330), an air outlet hole (333) is disposed on the top of the inner chamber of the second pipe (330), and the water outlet hole (331) and the air outlet hole (333) are coaxially disposed.

7. The fume extracting device of claim 6, wherein the bottom of the inner chamber of the second pipe (330) is gradually recessed downwardly in a direction of being close to the water outlet hole (331), and the fume extracting device further comprises a rotary plug (332) for closing the water outlet hole (331).

8. The fume extracting device of claim 5, wherein a side of the filtering cartridge (412) is provided with an air vent (414) to enable gases to enter the first pipe (320) from the filtering cartridge (412).

9. The fume extracting device of claim 5, wherein the volute fan (200) comprises a volute, a wind wheel and a motor, the volute is mounted above the second pipe (330) and fixed on a sidewall of the second pipe (330); the wind wheel is located inside the volute and the motor is located above the volute; a motor shaft of the motor faces downward to connect with the wind wheel.

10. An induction cooker integrated stove, comprising the fume extracting device mentioned in any one of claims 1 to 9.

## Patentansprüche

1. Wrasenabsaugvorrichtung zur Verwendung in einem integrierten Induktionsherd, umfassend:
einen Kastenkörper (100), wobei eine Montageöffnung (110) an einer oberen Stirnfläche des Kastenkörpers (100) angeordnet ist;
ein Spiralgehäusegebläse (200), das innerhalb des Kastenkörpers (100) angeordnet ist, wobei ein Lufteinlassende in einem mittleren Bereich des Spiralgehäusegebläses (200) nach unten weist;
ein Wrasenabführrohr (500), das mit einem Luftauslassende des Spiralgehäusegebläses (200) in Verbindung steht; und
ein Wrasenabsaugrohr (300), das innerhalb des Kastenkörpers (100) angeordnet ist, wobei ein Ende des Wrasenabsaugrohrs (300) nach oben durch die Montageöffnung (110) hindurchgeführt und auf dem Kastenkörper (100) angeordnet ist und das andere Ende des Wrasenabsaugrohrs (300) mit dem Lufteinlassende des Spiralgehäusegebläses (200) in Verbindung steht;
**dadurch gekennzeichnet, dass** sich das durch den Kastenkörper (100) hindurchgeführte Ende des Wrasenabsaugrohrs (300) horizontal erstreckt, um eine Stützbasis (310) zu bilden, eine Unterseite der Stützbasis (310) an der oberen Stirnfläche des Kastenkörpers (100) anliegt und daran befestigt ist, und eine Filterbaugruppe (400) innerhalb der Stützbasis (310) angeordnet ist, um Gase zu filtern, die von außen in das Wrasenabsaugrohr (300) eintreten.

2. Wrasenabsaugvorrichtung nach Anspruch 1, wobei die Filterbaugruppe (400) einen Filterbecher (410), ein Filtersieb (420), und eine Lufteinlassabdeckung (430) umfasst; ein Teil der Struktur des Filterbechers (410) durch die Montageöffnung (110) in das Wrasenabsaugrohr (300) eingeführt ist und ein Teil der Struktur des Filterbechers (410) an der Stützbasis (310) anliegt, sodass die Stützbasis (310) den Filterbecher (410) stützt; eine Öffnung eines oberen Endes des Filterbechers (410) mit einer Öffnung eines oberen Endes der Stützbasis (310) übereinstimmt, das Filtersieb (420) auf die Öffnung des oberen Endes des Filterbechers (410) aufgelegt ist, und das Filtersieb (420) mit der Lufteinlassabdeckung (430) abgedeckt ist.

3. Wrasenabsaugvorrichtung nach Anspruch 2, wobei der Filterbecher (410) ein Stützgestell (411) und eine Filterkartusche (412) umfasst und eine Seite des Stützgestells (411) mit einem oberen Ende der Filterkartusche (412) in Verbindung steht; ein unteres Ende des Stützgestells (411) an der Stützbasis (310) anliegt und die Filterkartusche (412) in das Wrasenabsaugrohr (300) eingeführt ist.

4. Wrasenabsaugvorrichtung nach Anspruch 3, wobei Anhebegriffe (413) an der Unterseite des Stützgestells (411) angeordnet sind.

5. Wrasenabsaugvorrichtung nach Anspruch 3, wobei das Wrasenabsaugrohr (300) ferner ein erstes Rohr (320) und ein zweites Rohr (330) umfasst; sich das erste Rohr (320) vertikal erstreckt; ein oberes Ende des ersten Rohrs (320) mit der Stützbasis (310) verbunden ist, ein unteres Ende des ersten Rohrs (320) mit einem Ende des zweiten Rohrs (330) verbunden ist, und ein anderes Ende des zweiten Rohrs (330) mit dem Lufteinlassende des Spiralgehäusegebläses (200) verbunden ist.

6. Wrasenabsaugvorrichtung nach Anspruch 5, wobei eine Wasserauslassöffnung (331) am Boden eines Innenraums des zweiten Rohrs (330) angeordnet ist, eine Luftauslassöffnung (333) an der Oberseite des Innenraums des zweiten Rohrs (330) angeordnet ist, und die Wasserauslassöffnung (331) und die Luftauslassöffnung (333) koaxial angeordnet sind.

7. Wrasenabsaugvorrichtung nach Anspruch 6, wobei der Boden des Innenraums des zweiten Rohrs (330) zur Wasserauslassöffnung (331) hin zunehmend nach unten vertieft ist und die Wrasenabsaugvorrichtung ferner einen Drehstopfen (332) zum Verschließen der Wasserauslassöffnung (331) umfasst.

8. Wrasenabsaugvorrichtung nach Anspruch 5, wobei an einer Seite der Filterkartusche (412) eine Luftdurchtrittsöffnung (414) angeordnet ist, um Gasen zu ermöglichen, aus der Filterkartusche (412) in das erste Rohr (320) einzutreten.

9. Wrasenabsaugvorrichtung nach Anspruch 5, wobei das Spiralgehäusegebläse (200) ein Spiralgehäuse, ein Laufrad, und einen Motor umfasst; das Spiralgehäuse oberhalb des zweiten Rohrs (330) montiert und an einer Seitenwand des zweiten Rohrs (330) befestigt ist; das Laufrad innerhalb des Spiralgehäuses angeordnet ist und der Motor oberhalb des Spiralgehäuses angeordnet ist; eine Motorwelle des Motors zur Verbindung mit dem Laufrad nach unten gerichtet ist.

10. Integrierter Induktionsherd, umfassend die Wrasenabsaugvorrichtung nach einem der Ansprüche 1 bis 9.

## Revendications

1. Dispositif d'extraction de fumée, appliqué à un cuiseur intégré à induction et comprenant :
un corps de boîte (100), un trou de montage (110) étant disposé sur une surface d'extrémité supérieure du corps de boîte (100) ;
un ventilateur à volute (200), situé à l'intérieur du corps de boîte (100), une extrémité d'entrée d'air à une partie de milieu du ventilateur à volute (200) étant orientée vers le bas ;
un tuyau d'évacuation de fumée (500), le tuyau d'évacuation de fumée (500) étant en communication avec une extrémité de sortie d'air du ventilateur à volute (200) ;
un tuyau d'extraction de fumée (300), situé à l'intérieur du corps de boîte (100), une extrémité du tuyau d'extraction de fumée (300) étant insérée vers le haut à travers le trou de montage (110) et disposée sur le corps de boîte (100), et l'autre extrémité du tuyau d'extraction de fumée (300) étant en communication avec l'extrémité d'entrée d'air du ventilateur à volute (200) ; **caractérisé en ce que**
l'extrémité du tuyau d'extraction de fumée (300) insérée à travers le corps de boîte (100) s'étendant horizontalement pour former une base de support (310), un côté inférieur de la base de support (310) s'appuyant contre et fixé sur la surface d'extrémité supérieure du corps de boîte (100), et un ensemble filtrant (400) étant disposé à l'intérieur de la base de support (310) pour filtrer des gaz entrant dans le tuyau d'extraction de fumée (300) de l'extérieur.

2. Dispositif d'extraction de fumée selon la revendication 1, dans lequel l'ensemble filtrant (400) comprend une coupelle filtrante (410), un tamis (420) et un couvercle d'entrée d'air (430), une partie de structure de la coupelle filtrante (410) est insérée à travers le trou de montage (110) jusqu'à l'intérieur du tuyau d'extraction de fumée (300), et une partie de structure de la coupelle filtrante (410) s'appuie contre la base de support (310) pour permettre à la base de support (310) de soutenir la coupelle filtrante (410), une ouverture d'une extrémité supérieure de la coupelle filtrante (410) correspond à une ouverture d'une extrémité supérieure de la base de support (310), le tamis (420) est posé sur l'ouverture de l'extrémité supérieure de la coupelle filtrante (410), et le couvercle d'entrée d'air (430) recouvre le tamis (420).

3. Dispositif d'extraction de fumée selon la revendication 2, dans lequel la coupelle filtrante (410) comprend un support en berceau (411) et une cartouche filtrante (412), et un côté du support en berceau (411) est en communication avec une extrémité supérieure de la cartouche filtrante (412), une extrémité inférieure du support en berceau (411) s'appuie contre la base de support (310), et la cartouche filtrante (412) est insérée à l'intérieur du tuyau d'extraction de fumée (300).

4. Dispositif d'extraction de fumée selon la revendication 3, dans lequel des poignées de levage (413) sont disposées au fond du support en berceau (411).

5. Dispositif d'extraction de fumée selon la revendication 3, dans lequel le tuyau d'extraction de fumée (300) comprend en outre un premier tuyau (320) et un second tuyau (330), le premier tuyau (320) s'étend verticalement, une extrémité supérieure du premier tuyau (320) est reliée à la base de support (310), une extrémité inférieure du premier tuyau (320) est reliée à une extrémité du second tuyau (330), et l'autre extrémité du second tuyau (330) est reliée à l'extrémité d'entrée d'air du ventilateur à volute (200).

6. Dispositif d'extraction de fumée selon la revendication 5, dans lequel un trou de sortie d'eau (331) est disposé au fond d'une chambre interne du second tuyau (330), un trou de sortie d'air (333) est disposé sur le sommet de la chambre interne du second tuyau (330), et le trou de sortie d'eau (331) et le trou de sortie d'air (333) sont disposés de manière coaxiale.

7. Dispositif d'extraction de fumée selon la revendication 6, dans lequel le fond de la chambre interne du second tuyau (330) se creuse progressivement vers le bas dans une direction s'approchant du trou de sortie d'eau (331), et le dispositif d'extraction de fumée comprend en outre un bouchon rotatif (332) destiné à fermer le trou de sortie d'eau (331).

8. Dispositif d'extraction de fumée selon la revendication 5, dans lequel un côté de la cartouche filtrante (412) est pourvu d'un orifice de ventilation (414) permettant aux gaz d'entrer dans le premier tuyau (320) à partir de la cartouche filtrante (412).

9. Dispositif d'extraction de fumée selon la revendication 5, dans lequel le ventilateur à volute (200) comprend une volute, une roue à aubes et un moteur, la volute est montée au-dessus du second tuyau (330) et fixée sur une paroi latérale du second tuyau (330) ; la roue à aubes est située à l'intérieur de la volute et le moteur est situé au-dessus de la volute ; un arbre moteur du moteur est orienté vers le bas pour être reliée à la roue à aubes.

10. Cuiseur intégré à induction, comprenant le dispositif d'extraction de fumée selon l'une quelconque des revendications 1 à 9.
